# EUROPÄISCHE PATENTSCHRIFT

(11) **EP 0 563 814 B1**
(45) Veröffentlichungstag und Bekanntmachung des Hinweises auf die Patenterteilung: **05.01.2000**
(21) Anmeldenummer: 93105011.6
(22) Anmeldetag: 26.03.1993
(51) Int. Cl.: C07D 473/32, A61K 31/52

(54) **Carbonsäureester von 2-Amino-7-(1,3-dihydroxy-2-propoxymethyl)purin, deren Herstellung sowie deren Verwendung als antivirales Mittel**
Carbonic acid esters of 2-amino-7-(1,3-dihydroxy-2-propoxymethyl)purines, their preparation and their use as antiviral agents
Esters d'acide carbonique de 2-amino-7-(1,3-dihydroxy-2-propoxyméthyl)purines, leur préparation et leur utilisation comme agents antiviraux

(30) Priorität: 28.03.1992 DE 4210221
(43) Veröffentlichungstag der Anmeldung: 06.10.1993
(73) Patentinhaber: HOECHST AKTIENGESELLSCHAFT, 65926 Frankfurt am Main (DE)
(72) Erfinder: Jähne, Gerhard, Dr., W-6230 Frankfurt am Main (DE); Helsberg, Matthias, Dr., W-6233 Kelkheim/Ts. (DE); Winkler, Irvin, Dr., W-6237 Liederbach (DE); Gross, Gerhard, Dr., W-6093 Flörsheim am Main (DE); Scholl, Thomas, Dr., W-5300 Bonn (DE)

(56) Entgegenhaltungen:
- EP-A- 0 452 680

## Beschreibung

Es wurde bereits gefunden (s. EP 452 680), daß substituierte Purine der Formel 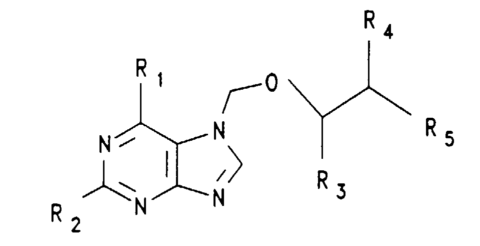 eine antivirale Wirkung aufweisen.

Es hat sich nun herausgestellt, daß bestimmte Carbonsäureester substituierter Purine eine besondere antivirale Wirksamkeit haben und darüber hinaus eine besonders ausgeprägte Bioverfügbarkeit nach oraler Verabreichung aufweisen.

Erfindungsgegenstand sind demzufolge Verbindungen der Formel 1 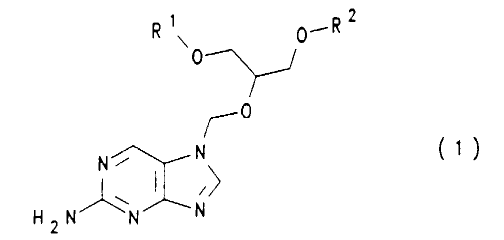 in denen die Reste R¹ und/oder R² unabhängig voneinander Acylreste der Formel

- C ( = O ) - R³ (2)

darstellen,
wobei R³ C₁-C₃-Alkyl bedeutet
sowie deren physiologisch verträgliche Salze.

Eine ganze besondere Bedeutung besitzt die obengenannte Verbindung der Formel 1, in der R³ Methyl bedeutet.

Die erfindungsgemäßen Verbindungen der Formel I können in der acyclischen Seitenkette ein oder mehrere chirale Zentren aufweisen. Die Verbindungen liegen in der Regel als Racemate vor; eine Herstellung bzw. Isolierung der reinen Enantiomeren ist möglich. Gegenstand der Erfindung sind deshalb sowohl die reinen Enantiomeren als auch Mischungen derselben, wie z.B. das zugehörige Racemat.

Besonders für therapeutische Zwecke geeignete Salze der erfindungsgemäßen Verbindungen sind Salze von physiologisch verträglichen Säuren wie Essigsäure, Milchsäure, Äpfelsäure, p-Toluolsulfonsäure, Methansulfonsäure, Isäthionsäure, Salzsäure, Schwefelsäure oder Phosphorsäure.

Weiterhin gehört zum Gegenstand der vorliegenden Erfindung ein Verfahren zur Herstellung von erfindungsgemäßen Verbindungen , das dadurch gekennzeichnet ist, daß man die Verbindung der Formel 1' 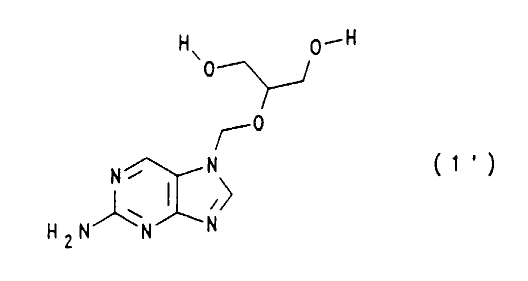 mit Carbonsäuren der Formel 3,

R⁴ - COOH (3)

wobei R⁴ C₁-C₃-Alkyl bedeutet, mit einem wasserentziehenden Mittel unter Beifügung einer Base in einem organischen Lösungsmittel unter Erhalt von Verbindungen der Formel 1 umsetzt und anschließend die Mono- und Diester voneinander mittels herkömmlicher Methoden trennt.

Die obengenannte Verbindung der Formel 1' läßt sich z.B. wie in der EP 452 860 beschrieben, herstellen.
Ein bevorzugtes wasserentziehendes Mittel zur Durchführung der obengenannten Reaktion ist z.B. Dicyclohexylcarbodiimid. Als Basen eignet sich z.B. N,N'-Dimethylaminopyridin bzw. dessen Derivate.

Geeignete organische Lösungsmittel zur Durchführung der Reaktion sind z.B. Dimethylformamid, N-Methyl-2-pyrrolidon, Pyridin, Dichlormethan, 1,2-Dichlorethan oder Tetrahydrofuran.

Zur Durchführung der Reaktion wird vorzugsweise die Verbindung der Formel 1' in dem organischen Lösungsmittel gelöst oder suspendiert und vorzugsweise bei-10°C bis +40°C insbesondere bei Raumtemperatur, unter Rühren nacheinander vorzugsweise mit mindestens 2 Äquivalenten insbesondere 2,2-3 Äquivalenten, der entsprechenden Carbonsäure, gegebenenfalls in dem entsprechenden Lösungsmittel gelöst, katalytischen Mengen, vorzugsweise 0,2-0,4 Äquivalenten, N,N-Dimethylaminopyridin, und vorzugsweise wenigstens 2 Äquivalenten, insbesondere 3-5 Äquivalenten, Dicyclohexylcarbodiimid versetzt und anschließend die Mischung 1-24 Stunden, vorzugsweise 6-10 Stunden, bei -10°C bis +50°C, vorzugsweise bei Raumtemperatur bis +40°C, gerührt. Zur Vervollständigung der Reaktion werden gegebenenfalls nach dieser Zeit ein weiteres Äquivalent der entsprechenden Carbonsäure, 0,2 Äquivalente N,N-Dimethylaminopyridin und 2 Äquivalente Dicyclohexylcarbodiimid zugegeben und weitere 5-24 Stunden, vorzugsweise 5-10 Stunden gerührt. Danach wird vom ausgefallenen Dicyclohexylharnstoff abfiltriert und das erfindungsgemäße Produkt isoliert. Die Isolation erfolgt z.B. chromatographisch oder durch Kristallisation, vorzugsweise - nach Einengen des Filtrats - durch Chromatographie z.B. über Kieselgel mit z.B. Dichlormethan/Methanol 9/1.

Die Monoester lassen sich gewinnen, indem man die Reaktion dünnschichtchromatographisch verfolgt und vorzeitig abbricht und danach wie oben beschrieben z.B. chromatographisch reinigt.

Die gegebenenfalls nötige Isolierung von optisch aktiven Verbindungen erfolgt nach Methoden des Standes der Technik.

Zum Erfindungsgegenstand gehört weiterhin die Verwendung der erfindungsgemäßen Verbindungen der Formel 1 als antivirale Mittel zur Behandlung oder Prophylaxe viraler Erkrankungen.

Besonders wirksam sind die erfindungsgemäßen Verbindungen gegen Herpes Simplex Virus Typ 1, Herpes Simplex Virus Typ 2, humanes Cytomegalie-Virus, murines Cytomegalie-Virus, Varicella Zoster Virus, Epstein Barr Virus und humanes Herpes Virus 6 (HHV-6).

Des weiteren betrifft die vorliegende Erfindung Arzneimittel mit einem Gehalt an mindestens einer erfindungsgemäßen Verbindung.

Die erfindungsgemäßen Arzneimittel können bevorzugt enteral (oral) aber auch parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0,1-10, vorzugsweise 0,2-8 mg/kg Körpergewicht verabreicht. Sie werden zweckmäßig in Dosierungseinheiten verabreicht, die mindestens die wirksame Tagesmenge der erfindungsgemäßen Verbindungen z.B. 30-300, vorzugsweise 50-250 mg enthalten.

Wirkung gegen systemische Herpes simplex 1 Virus (HSV-1) Infektion: NMRI-Mäuse, spezifisch pathogenfrei, im Gewicht von 15-18 g wurden intraperitoneal mit HSV-1 infiziert und anschließend mit den erfindungsgemäßen Verbindungen oral therapiert. Die Behandlung erfolgte erstmals 3 Stunden nach der Infektion und wurde zweimal täglich über 4 Tage fortgesetzt. Der Behandlungserfolg wurde anhand des Krankheitsverlaufes und der Überlebensrate gegenüber der unbehandelten Infektionskontrolle bestimmt. Letztere erhielt anstelle der erfindungsgemäßen Verbindungen physiologische Kochsalzlösung appliziert. Die Beobachtungszeit betrug zwei Wochen. Tabelle 1 zeigt die Ergebnisse dieser Untersuchung.

**Tabelle 1**

| Antivirale Wirkung gegen HSV-1 in der NMRI-Maus bei oraler Verabreichung | | | |
|---|---|---|---|
| Beispiel | Dosierung (µmol/kg) | mittlere Überlebenszeit (Tage) | Überlebende/Gruppengröße |
| 1 | 9 x 10 | > 14 Tage | 5/5 |
| | 9 x 30 | > 14 Tage | 5/5 |
| | 9 x 100 | > 14 Tage | 5/5 |
| | | | |
| 2 | 9 x 30 | | 5/5 |
| | 9 x 100 | | 5/5 |
| | | | |
| 3 | 9 x 30 | | 5/5 |
| | 9 x 100 | | 5/5 |
| | | | |
| Formel 1, | 9 x 10 | 10.5 ± 0.7 | 3/5 |
| R ¹ =R ² =H (= Verbindung der Formel 1') | 9 x 30 | 9.5 ± 1.3 | 1/5 |
| | 9 x 100 | | 5/5 |
| | | | |
| Kontrolle | 9 x 10 | 7.8 ± 1.1 | 1/5 |

### Bestimmung der oralen Bioverfügbarkeit:

Rhesusaffen wurden 24 Stunden vor Versuchsbeginn in Einzelkäfige gesetzt. 12 Stunden vor Versuchsbeginn wurde das Futter entzogen, während Trinkwasser vor und während des Versuches unbeschränkt zur Verfügung stand. Je zwei Tiere erhielten die erfindungsgemäßen Verbindungen, ein Tier die Verbindung der Formel 1', jeweils 105 µmol/kg, mittels einer Magensonde in einem Volumen von 5 ml Leitungswasser appliziert. Mit weiteren 20 ml Wasser wurde nachgespült.

Ein Tier erhielt 42 µmol/kg der Verbindung der Formel 1', in einer Konzentration von 10 mg/ml in physiologischer Kochsalzlösung in eine Oberarmvene injiziert.

Bei allen Tieren wurden zu verschiedenen Zeitintervallen bis 24 Stunden nach Substanzapplikation Blutproben aus der Oberschenkelvene entnommen.

Das Blut wurde bei 4°C bis zur Gerinnung aufbewahrt und anschließend zentrifugiert. Das daraus gewonnene Serum wurde bei -20°C bis zur Analyse der Proben aufbewahrt. 200 µl Serum wurden in ein Reaktionsgefäß vorgelegt und mit 100 ml Wasser verdünnt. Nach Zugabe von 30 µl 50 %iger Trichloressigsäure wurde am ^{(R)}Vortex-Mixer durchmischt und anschließend 3 Minuten zentrifugiert. 50 µl der überstehenden Lösung wurden mittels HPLC untersucht.
Chromatographische Trennung: Säule ^{(R)}Nucleosil C₁₈, 125 x 4,6 mm; Mobile Phase: Phosphorsäure, 0.01 molar; Fluß: 1.0 ml/min; Detektion: Fluoreszenz, Ex: 310 nm, Em: 360 nm; Retentionszeit: 3,5 min (Verbindung der Formel 1, wobei R¹ = R² = H ist, d.h. Verbindung der Formel 1').

Die Berechnung erfolgte über die Auswertung der Fläche unter den Daten (AUD). Die metabolische Verfügbarkeit der Verbindung der Formel 1', nach oraler Gabe der Verbindung des Beispiels 1 betrug im Mittel 37,80 % (34,47 % und 41,16 %). Bei Gabe der Verbindung des Beispiels 2 wurde eine Bioverfügbarkeit der Verbindung der Formel 1' von im Mittel 20,11 % (23,73 % und 16,50 %) erreicht (die genannten Verbindungen werden im Körper zu Verbindungen der Formel 1' metabolisiert).
Die Bioverfügbarkeit bei oraler Gabe der Verbindung der Formel 1' beträgt hingegen im Mittel nur 15 %.

### Bestimmung der oralen Bioverfügbarkeit (Maus):

Folgende Bioverfügbarkeiten der Verbindungen der Formel 1' wurden bei Mäusen nach oraler Gabe der erfindungsgemäßen Verbindungen ermittelt:

| Verbindung | Applikationsart | Dosis [mg/kg] | AUD (0-->oo) [(µgxmin)/ml] | Bioverfügbarkeit | [%] |
|---|---|---|---|---|---|
| Formel 1' | i.v. | 25 | 13.39 | | 100 |
| | p.o. | 100 | 9.23 | 17.2 | |
| Beispiel 1 | i.v. | 25 | 13.21 | 98.6 | |
| | p.o. | 100 | 27.32 | 51.1 | |
| Beispiel 2 | p.o. | 100 | 36.03 | 67.3 | |
| Beispiel 3 | p.o | 100 | 38.40 | 71.7 | |

Durch die nachfolgenden Ausführungsbeispiele und durch den Inhalt der Patentansprüche wird die vorliegende Erfindung näher erläutert.

### Beispiele

1. Verbindung der Formel 1, wobei R¹ = R² = Acetyl ist;
   1,2 g (5 Mmol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680 A beschrieben) werden in 50 ml wasserfreiem Dimethylformamid gelöst. Man gibt nacheinander 0,63 ml (11 mMol) Essigsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,32 ml (5,5 mMol) Essigsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,51 g (93,5 % d.Th.) 2-Amino-7-[(1,3-bis-acetoxy-2-propoxy)methyl]purin mit dem Schmelzpunkt 158°C als farblose Kristalle. ¹H-NMR (270 Mhz, d₆-dmso) δ [ppm]: 8,65 (s,1H), 8,43 (s,1H), 6,27 (s,2H), 5,68 (s,2H), 4,15-3,86 (m,5H), 1,83 (s,6H).
2. Verbindung der Formel 1, wobei R¹ = R² = Butanoyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680A beschrieben) werden in 50 ml wasserfreiem Dimethylformamid gelöst. Man gibt nacheinander 1,0 ml (11 mMol) Buttersäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,5 ml (5,5 mMol) Buttersäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,84 g (97,1 % d.Th.) 2-Amino-7-[(1,3-bis-butyryloxy-2-propoxy)methyl]purin mit dem Schmelzpunkt 123°C als farblose Kristalle.
   ¹H-NMR (270 MHz, d₆-dmso) δ [ppm]: 8,64 (s,1H), 8,43 (s,1H), 6,27 (s,2H), 5,67 (s,2H), 4,18-3,79 (m,5H), 2,05 (t,4H), 1,42 (m,4H), 0,83 (t,6H).
3. Verbindung der Formel 1, wobei R¹ = R² = Propanoyl ist:
   1,2 g (5 mMol) 2-Amino-7-[(1,3-dihydroxy-2-propoxy)methyl]purin (Verbindung der Formel 1, wobei R¹ = R² = Wasserstoff ist; hergestellt wie in der Europäischen Patentanmeldung EP 452 680 A beschrieben) werden in 50 ml wasserfreiem Dimethylformamid gelöst. Man gibt nacheinander 0,82 ml (11 mMol) Propionsäure, 0,2 g (1,6 mMol) N,N-Dimethylaminopyridin und 3,96 g (19 mMol) N,N'-Dicyclohexylcarbodiimid zu und rührt die Mischung 6 Stunden bei Raumtemperatur. Nach dieser Zeit gibt man erneut 0,41 ml (5,5 mMol) Propionsäure, 0,1 g (0,8 mMol) N,N-Dimethylaminopyridin und 1,98 g (9,5 mMol) N,N'-Dicyclohexylcarbodiimid zu und rührt weitere 17 Stunden bei Raumtemperatur. Ausgefallener N,N'-Dicyclohexylharnstoff wird abfiltriert, das Filtrat im Vakuum eingeengt und der Rückstand über Kieselgel mit Dichlormethan/Methanol 9/1 als Laufmittelgemisch chromatographiert. Man erhält 1,53 g (87,2 % d.Th.) 2-Amino-7-[(1,3-bis-propionyloxy-2-propoxy)methyl]purin mit dem Schmelzpunkt 143°C als farblose Kristalle.
   ¹H-NMR (270 MHz, d₆-dmso) δ [ppm]: 8,64 (s,1H), 8,42 (s,1H), 6,28 (s,2H), 5,67 (s,2H), 4,13 (m,2H), 4,05-3,89 (m,3H), 2,10 (q,4H), 0,92 (t,6H).

## Patentansprüche

1. Verbindungen der Formel I 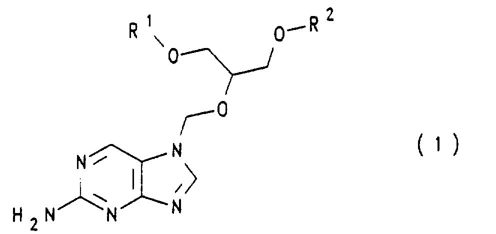 in denen die Reste R¹ und/oder R² unabhängig voneinander Acylreste der Formel
-C(=O)-R³ (2)
darstellen,
wobei R³ C1-C3-Alkyl bedeutet
sowie deren physiologisch verträgliche Salze.

2. Verbindungen der Formel 1 gemäß Anspruch 1, dadurch gekennzeichnet, daß R³ Methyl bedeutet.

3. Verfahren zur Herstellung von Verbindungen der Formel 1 gemäß den Ansprüchen 1 oder 2, dadurch gekennzeichnet, daß man die Verbindung der Formel 1', 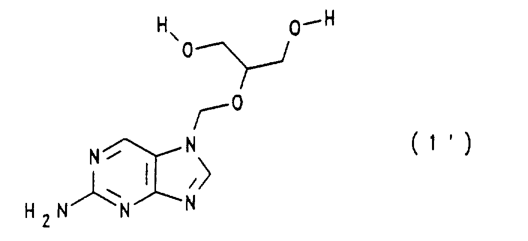 mit Carbonsäuren der Formel 3,
R⁴-COOH (3)
wobei R⁴ C1-C3-Alkyl bedeutet, mit einem wasserentziehenden Mittel wie N,N'-Dicyclohexylcarbodiimid unter Beifügung einer Base wie N,N-Dimethylaminopyridin in einem Lösungsmittel wie Dimethylformamid unter Erhalt von Verbindungen der Formel 1 umsetzt.

4. Verbindungen der Formel 1 gemäß den Ansprüchen 1 oder 2 zur Anwendung als antivirales Mittel.

5. Verbindungen der Formel 1 gemäß Ansprüchen 1 oder 2 zur Anwendung als Antiherpesmittel.

6. Arzneimittel mit einem Gehalt an mindestens einer Verbindung der Formel 1 gemäß den Ansprüchen 1 oder 2.

7. Verwendung von Verbindungen der Formel 1 gemäß den Ansprüchen 1 oder 2 zur Herstellung von Arzneimitteln zur Behandlung von Viruserkrankungen.

8. Verfahren zur Herstellung von Arzneimitteln gemäß Anspruch 6, dadurch gekennzeichnet, daß man mindestens eine Verbindung der Formel 1 gemäß den Ansprüchen 1 oder 2 gegebenenfalls mit geeigneten Hilfs- und/oder Trägerstoffen in eine geeignete Darreichungsform bringt.

## Claims

1. A compound of the formula I 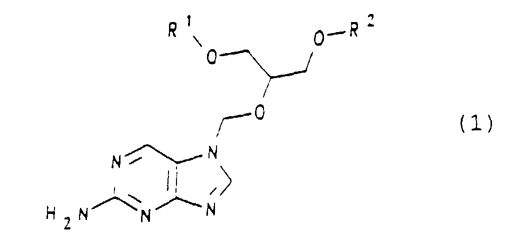 in which the radicals R¹ and/or R², independently of each other, are acyl radicals of the formula
-C(=O)-R³ (2),
where R³ is (C₁-C₃)-alkyl, and its physiologically tolerated salts.

2. A compound of the formula 1 as claimed in claim 1, wherein R³ is methyl.

3. A process for preparing compounds of the formula 1 as claimed in claim 1 or 2, wherein the compound of the formula 1' 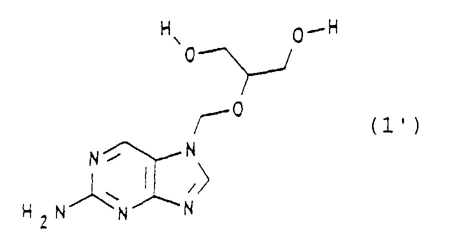 is reacted with carboxylic acids of the formula 3
R⁴-COOH (3)
where R⁴ is (C₁-C₃)-alkyl, in a solvent, such as dimethylformamide, in the presence of a dehydrating agent, such as N,N'-dicyclohexylcarbodiimide, and with the addition of a base, such as N,N-dimethylaminopyridine, to give compounds of the formula 1.

4. A compound of the formula 1 as claimed in claim 1 or 2 for use as an antiviral agent.

5. A compound of the formula 1 as claimed in claim 1 or 2 for use as an anti-herpes agent.

6. A pharmaceutical containing at least one compound of the formula 1 as claimed in claim 1 or 2.

7. The use of compounds of the formula 1 as claimed in claim 1 or 2 for preparing pharmaceuticals for treating viral diseases.

8. A process for preparing pharmaceuticals as claimed in claim 6, wherein at least one compound of the formula 1 as claimed in claim 1 or 2 is converted into a suitable form for administration, where appropriate with suitable auxiliary substances and/or excipients.

## Revendications

1. Composé de la formule 1 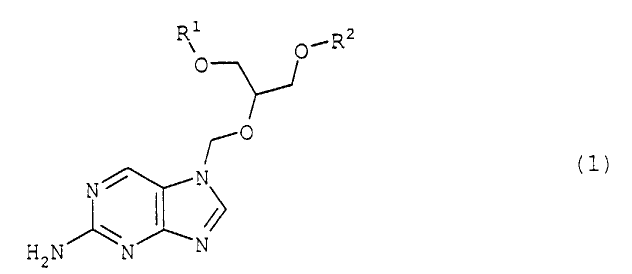 dans laquelle les résidus R¹ et/ou R² représentent indépendamment l'un de l'autre des résidus acyle de formule
-C(=O)-R³ (2)
dans laquelle R³ signifie un alkyle en C₁ à C₃, ainsi que leurs sels physiologiquement acceptables.

2. Composé de la formule 1 selon la revendication 1, caractérisé en ce que R³ signifie le méthyle.

3. Procédé de préparation de composés de la formule 1 selon la revendication 1 ou la revendication 2, caractérisé en ce que l'on fait réagir le composé de la formule 1' 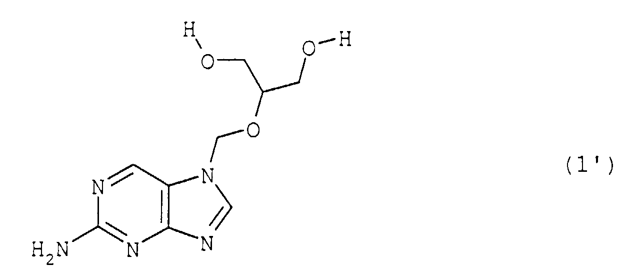 avec des acides carboxyliques de la formule 3
R⁴-COOH (3)
dans laquelle R⁴ désigne un groupe alkyle en C₁ à C₃, avec un agent déshydratant tel que le carbodiimide de N,N'-dicyclohexyle, en ajoutant une base telle que la N,N-diméthylaminopyridine, dans un solvant tel que le diméthylformamide, pour obtenir le composé de la formule 1.

4. Composés de la formule 1 selon la revendication 1 ou la revendication 2, destiné à être utilisés comme agents antiviraux.

5. Composés de la formule 1 selon la revendication 1 ou la revendication 2, destiné à être utilisés comme agents anti-herpétiques.

6. Médicaments avec une teneur en au moins un composé de la formule 1 selon la revendication 1 ou la revendication 2.

7. Utilisation de composés de la formule 1 selon la revendication 1 ou la revendication 2 pour la fabrication de médicament destinés au traitement de maladies virales.

8. Procédé de préparation de médicaments selon la revendication 6, caractérisé en ce que l'on amène au moins un composé de la formule 1 selon la revendication 1 ou la revendication 2 sous une forme posologique appropriée, le cas échéant avec des additifs et/ou des véhicules appropriés.
